Europäisches Patentamt

European Patent Office

Office européen des brevets

(11) Publication number: **0 365 459**
**A1**

## (12) EUROPEAN PATENT APPLICATION

(21) Application number: 89500096.6

(51) Int. Cl.5: **A61B 13/00**

(22) Date of filing: 06.10.89

(30) Priority: **10.10.88 ES 8802988**

(43) Date of publication of application:
**25.04.90 Bulletin 90/17**

(84) Designated Contracting States:
**AT BE CH DE FR GB GR IT LI LU NL SE**

(71) Applicant: **Garrido-Lestache y Cabrera, Antonio**
**Hermosilla 81**
**E-28001 Madrid(ES)**

(72) Inventor: **Garrido-Lestache y Cabrera, Antonio**
**Hermosilla 81**
**E-28001 Madrid(ES)**

(74) Representative: **Munoz Garcia, Antonio**
**Miguel Angel 16, 2. drcha.**
**E-28010 Madrid(ES)**

(54) **Tongue depressor for pediatrics use.**

(57) A tongue depressor for use in pediatrics of those include under the quality of disposable,essentially characterised by the fact that it is constituted from a main body (1) having a cylindrical configuration,made in a thermoplastic or similar material,fitted on one of its end with a butt (2) or appendage for its handling,while on the other end there is a spatulate elongation in slanted situation (3), with a configuration without edges,reinforced by means of a rib (4) produced in the same material.

FIG.-2

## TONGUE DEPRESSOR FOR PEDRIATICS USE

### SUBJECT OF INVENTION

This present invention refers to a tongue depressor for pedriatics use the obvious aim of which is to enable the physician to have means for exploring the baby's mouth, according with the newborn measurements, while substantially avoiding any eventual alteration to the patient throughout the exploration.

### SCOPE OF INVENTION

This invention finds its application within the surgery devices manufacturing industry, specially among those devoted to the manufacture of surgery material for medical checking and exploration.

### BACKGROUNDS OF INVENTION

It is well known that the main task of a tongue depressor is aimed to effect the patient's togue depression at time of undertaking his/her mouth's inner cavity checking.

At the same time that checking the mouth cavity contexts is performed, it is required, when performing medical examination of a newborn individual, not only to limit onself to the exploration of the patient's throat relative area and, consequently, using the conventional tongue depressor is justified, but it is simultaneously required to undertake an in-deep examination of the whole mouth cavity, including gums and their adjacent areas, thus becoming necessary to raise the lips in order to make this checking as exhaustive as possible.

However, up to this time there is no device available which while allowing to be applied for the tongue depression technique, may be used as well, given its specifications and measurements, for performing the mouth cavity total examination in a newborn individual when the specialist physician starts performing a careful medical examination thereof.

It is known the existence of the Spanish Utility Model number 171,101 which, being referred to a tongue depressor is essentially characterized because it is constituted from a rigid or semi-rigid material in a plastic or thermoplasic substance which has a resiliency making it capable to avoid arching or breakage and which, in its opposed end to the area thorugh which it is handled, shows a series of grooves excavated in transversal form, as for instance caramel, with which the infant pacient opposes lower resistance to being examined, since he has the absolute conviction that once the medical examination is over, he will be rewarded by the physician with the deviced covered with caramel for its consumption in full.

The above-mentioned model is practically identical to the small wooden rods or plates which being of one only use are being used in general by specialists.

It has been applied for, also in Spain, another Utility Model, number 217,823 which, also referred to a tongue depressor is constituted by a flashlight handle and a spatule incorporated having a replaceable character, which is characterized by the fact that the said flashlight is rechargeable by previously connecting it to the mains network and that it has been designed to incorporate a bulb in a housing.

The replaceable spatule has been designed in curved shape with an "H" transversal profile taking the form of a fork in its lower shape.

The Spanish Utility Model number 235,440 covers under its application a tongue depressor for throat examination characterized by the fact that it is constituted by a wire or ribs frame having a rigid contexture having on one of its ends a spatular configuration part, constituting a housing for a candy pastille which actually constitutes the depressor part becoming in contact with the tongue, while on the opposes end of the rib frame there is a holder for handling the depressor itself.

As may be inferred, although its configuration is different, the application and reasoning is exactly the same as for the above-mentioned model, in order to make easier the physician's task when he has to perform an exploration of the infant patient's throat.

Under Utility Model number 262,754, which has also been applied for in Spain, it is covered a mouth-and-teeth exploration device, which is actually a modification performed in a toothbrush with its relevant bristles assembled on one face of the toothbrush head itself, incorporating in the brush handle a reflecting surface constituted by means of a mirror with its corresponding frame, the brush being provided with an articulation which allows multiple positions of the brush handle and head to effect a concrete dental exploration, thus being inneficient to perform throat exploration.

Under Utility Model number 269,043 it is applied for a tongue depressor having an elongated central handle and in each of its ends it has a sheet having a plain surface destined to come in contact with the tongue, each of the extreme sheets having different widths and with the surface textured for better holding ono the tongue.

The shape of each of these sheets is actually spatular with its vertex rounded and a plurality of depressions.

There are also applications, of course, for a series of medical instruments which having initially its application as laringoscopes, may also serve as depressors, although their main function is to perform explorations, picking up throat secretions or either for specific treatments.

As may be concluded from the above discussion, there is not, for the time being, any device to effect tongue depression which may be used, at the same time, to perform mouth exploration in a newborn by being specially shaped for such a function, thus being absolutely necessary to have available an idoneous instrument.

That notwithstanding, up to this time, it is not known the conceptual existence of a device identified with this subject.

## INVENTION DESCRIPTION

The tongue depressor for pediatrics proposed by this invention constitutes an exremely simple solution, therefore being of low cost, easily implantable within the circle wherein it should be used, offering optimous sanitary guarantees, allowing its easy elimination at time of being rejected.

To this end and in a more concrete manner, the tongue depressor for pediatrics use being proposed, is constituted from one only part body, preferentially moulded in plastic material which adopts the configuration of a cylindrical bar with no profiles, having the adequate dimensions to the end sought for, which has in one of its ends a handle or holder to help in handling it, while on the opposed end there is a plain elongation which adopts a circular configuration being slightly slanted in regard with the device main body.

In such a way that when the physician prepares himself to perform an exploration he may have available an element having small dimensions, perfectly sterilized, having the necessary specifications to perform the exploratory function inside a newborn's mouth and which, at the same time, has a handle with which the function to be performed by the physician is perfectly potentiated.

## DRAWING DESCRIPTION

To complete the description being made and in order to help to better understand the invention characteristics, this present specification is accompanied, forming an integral part therewith, by only one drawing sheet on which with illustrative non-limitative character has been represented the following.

Figure number 1 shows a plan view of the subject of this present invention.

Figure number 2 shows a side view of the same subject.

Finally, figure number 3 shows a view in which the handle of the subject has been sectioned, thus enabling to appreciate in perspective the depressing blade of the device in question.

## INVENTION PREFERENTIAL PERFORMANCE

In the light of the above figures, it may be noted that the tongue depressor for use in pediatrics being proposed is constituted from a one part body (1) which, as it has been said above, will be preferentially obtained by moulding based on a plactic material, a one part body which adopts the shape of a cylindrical bar there existing no edges on any kind through the whole complex, which has in one of its ends a thickened area with plain faces linked onto the general body, which has been references (2).

This area is devoted to help in handling the tongue depressor itself on the part of the physician.

In the end opposed to that of the area (2) there is an enlargement in circular shape, with perfectly rounded edges and plain faces which is slightly slanted in regard with the device central body, which has been referenced (3).

In order to impart a higher consistency to this spatular elongation it has been reinforced with some ribs (4) which hold it firmly attached to its trunk.

It is not deemed necessary to offer a longer description for any expert in this field to understand the scope of this invention as well as the advantages obtained thefrom.

All materials, shape, size and disposition of elements will be susceptible of variation, providing this does not imply any alteration in the invention essentials.

All terms used to describe this specification should be taken at all times in their ample non-limitative sense.

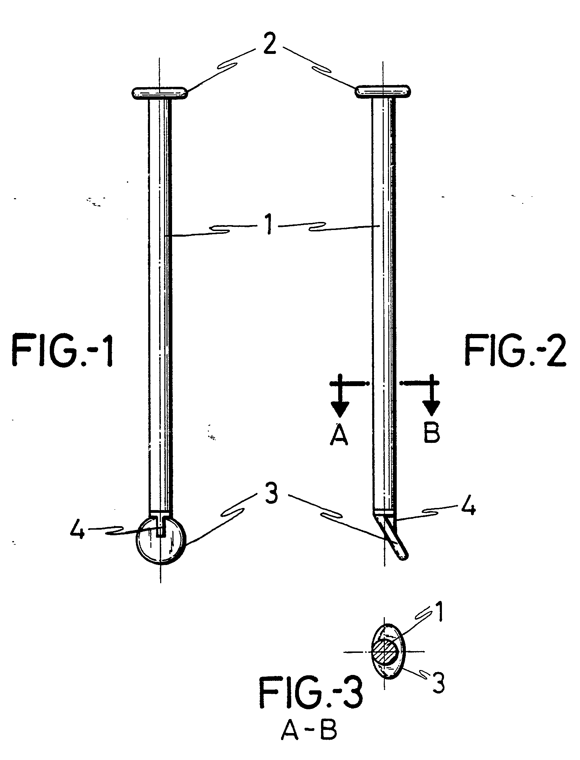

## Claims

1. A tongue depressor for use in pediatrics of those included under the quality of disposable, essencially characterized by the fact that it is constituted from a main body having a cylindrical configuration, made in a thermoplastic or similar material, fitted on one of its ends with a butt or appendage for its handling, while on the other end there is a spatulate elongation in slanted situation, with a configuration without edges, reinforced by means of a rib produced in the same material.

FIG.-1

FIG.-2

FIG.-3

A-B

European Patent
Office

**EUROPEAN SEARCH REPORT**

Application Number

EP 89 50 0096

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | FR-A-2 146 658 (Lerigner-Brodeur)<br>* Page 2, lines 26-38; fig 1 *<br>--- | 1 | A 61 B 13/00 |
| Y | US-A-3 768 477 (Anders et al.)<br>* Column 2, lines 40-55; fig 2 *<br>--- | 1 | |
| A | US-A-3 867 927 (Hergott)<br>* Column 2, lines 43-61; fig 1,2 *<br>----- | 1 | |
| | | | **TECHNICAL FIELDS SEARCHED (Int. Cl.5)**<br><br>A 61 B |

The present search report has been drawn up for all claims

| Place of search | Date of completion of the search | Examiner |
|---|---|---|
| THE HAGUE | 16-01-1990 | MOERS R.J. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................
& : member of the same patent family, corresponding
    document

EPO FORM 1503 03.82 (P0401)